# EUROPÄISCHE PATENTANMELDUNG

(11) **EP 1 104 677 A2**
(43) Veröffentlichungstag der Anmeldung: **06.06.2001**
(21) Anmeldenummer: 00122132.4
(22) Anmeldetag: 12.10.2000
(51) Int. Cl.: A61K 47/48

(54) **Neue Proteinkonjugate und Verfahren zu deren Herstellung**

(30) Priorität: 01.12.1999 DE 19957916
(71) Anmelder: Aventis Research & Technologies GmbH & Co. KG, 65926 Frankfurt am Main (DE)
(72) Erfinder: Kretzschmar, Gerhard, Dr., 65760 Eschborn (DE); Ziegler, Thomas, Prof. Dr., 50667 Köln (DE); Gerling, Sonja, 51063 Köln (DE); Lang, Martin, 72108 Rottenburg am Neckar (DE)
(74) Vertreter: Ackermann, Joachim, Dr.

(57) **Zusammenfassung**

Die Erfindung betrifft neue Proteinkonjugate der Formeln (la-c). Die Erfindung betrifft ferner ein Verfahren zur Herstellung der Proteinkonjugate durch Umsetzung von Proteinen mit drei niedermolekularen Komponenten in einem einzigen Reaktionsschritt, wobei simultan bis zu vier Reportergruppen oder Liganden in definierter Stöchimetrie und molekularem Abstand eingeführt werden, sowie Anwendungen der neuen Proteinkonjugate.

## Beschreibung

Die Erfindung betrifft neue Proteinkonjugate und Verfahren zu deren Herstellung. Die neuen Proteinkonjugate setzen sich zusammen einerseits aus einem natürlich vorkommenden, synthetisch modifizierten oder rekombinanten Protein, Enzym, Immunoglobulin, Antikörper, Rezeptorprotein oder Lectin, sowie andererseits aus gleichzeitig drei weiteren niedermolekularen Verbindungen aus der Gruppe der Amine, Carbonsäuren, Isonitrile, Aldehyde oder Ketone. Die Erfindung betrifft ferner ein Verfahren zur Herstellung der neuen Proteinkonjugate durch Umsetzung eines Proteins mit den drei niedermolekularen Komponenten in einem einzigen Reaktionsschritt, wobei simultan bis zu vier Reportergruppen oder Liganden in definierter Stöchimetrie und molekularem Abstand eingeführt werden, sowie Anwendungen der so erhältlichen Proteinkonjugate.

### Hintergrund:

Verknüpfungsprodukte aus Proteinen und niedermolekularen Verbindungen, sogenannte Proteinkonjugate, finden vielfältige Anwendungen im Bereich der "Life Sciences". In der Medizin gehören dazu beispielsweise therapeutisch einsetzbare Proteinkonjugate z.B. aus einem Antikörper und einem Cytostatikum. In der Diagnostik und Biochemie finden Proteinkonjugate vielfältige Einsatzgebiete zur Erkennung, Markierung und Quantifizierung der Proteine selbst oder ihrer molekularen Rezeptoren, Botenstoffe und Gegenspieler. In der medizinischen Immunologie werden Proteinkonjugate insbesondere aus Kohlenhydrat- und Nucleinsäure-Antigenen zur Immunisierung (Vaccinierung) verwendet. Das Protein kann einerseits als Vehikel zur Präsentation von Antigenen und zum zielgerichteten Transport einer oder mehrerer niedermolekularer Verbindungen dienen; andererseits kann der niedermolekulare Teil der Proteinkonjugate auch zur gezielten Modifizierung der Eigenschaften der Proteine selbst herangezogen werden. Die zuletzt genannte Variante umfaßt beispielsweise die gezielte Modifizierung der physikalischen Eigenschaften des Proteins, beispielsweise der thermischen Stabilisierung oder Löslichkeitsmerkmale eines katalytisch aktiven Enzyms, katalytisch aktiven Antikörpers (Abzym), therapeutisch wirksamen Proteins bzw. Antikörpers, oder die gezielte Modifizierung der eigentlichen katalytischen Aktivität, Spezifität oder der generellen Eigenschaften zur Erkennung eines Substrates, Rezeptors oder Liganden. Die Funktion des Proteinteils der Proteinkonjugate kann also vom bloßen Vehikel (Prodrug) zum zielgerichteten Transport (Drug Targeting) oder zur Ligandenpräsentation (Vaccine) bis zur katalytischen Aktivität oder der eigentlichen molekularen Erkennung reichen. Umgekehrt reicht die Funktion des "niedermolekularen" Teils der Proteinkonjugate von der physikalischen Modifizierung oder Stabilisierung über die Markierung mit einer oder mehreren charakteristischen und analytisch quantifizierbaren Reportergruppen (Diagnostika) bis zum eigentlichen Wirkprinzip (z.B. in Prodrugs) oder multivalenten Liganden (z.B. in Vaccinen).

Die meisten physiologisch aktiven Proteine liegen bereits als Proteinkonjugate vor, insbesondere als Konjugate mit Kohlenhydraten (Glycoproteine), was die biologische Bedeutung der Kohlenhydrate als Bindungspartner mit Proteinen unterstreicht. Sie werden vorwiegend auf der Proteinoberfläche über Serin, Threonin und Asparagin gebunden und sind beispielsweise an der Zell-Erkennung beteiligt. Die artifizielle Anbindung niedermolekularer Strukturen, insbesondere auch weiterer Kohlenhydrate zu sogenannten Neoglycoproteinen, kann z.B. zur gezielten Modulierung der Zellerkennung und zum Studium der zugrunde liegenden biologischen Phänomene herangezogen werden.

Das verfügbare Methodenrepertoire zur Anknüpfung von Kohlenhydraten, Wirkstoffen, Reportermolekülen, Farbstoffen, Antigenen, Liganden etc. an Proteine basiert im wesentlichen auf der Präsenz reaktiver Amino-, Carboxylat-, Thiol- oder Aldehydgruppen (nach Oxidation) in Proteinen. So ist bereits bekannt, daß Proteine, die freie Amino-, Carboxylat- oder Aldehydgruppen tragen, mit niedermolekularen Verbindungen, die ihrerseits freie Carboxylat- oder Aminogruppen tragen, zu Protein-Konjugaten verknüpft werden können (Advances in Carbohydrate Chemistry and Biochemistry *37*, 225-281, 1980). Freie Aminogruppen von Proteinen sind insbesondere die N-Termini der Proteine und die ε-Aminogruppen der Lysine, die sich zumindest teilweise mit niedermolekularen Carbonsäuren über eine Amidierungsreaktion verknüpfen lassen. Freie Carboxylatgruppen von Proteinen sind insbesondere die C-Termini der Proteine und die Carboxylatgruppen der Aminosäuren Asparaginsäure und Glutaminsäure, die zumindest teilweise mit niedermolekularen Aminen über eine Amidierungsreaktion verknüpft werden. Freie Thiolgruppen werden durch Cysteine präsentiert; freie Aldehydgruppen von Proteinen sind insbesondere oxidierte Kohlenhydratbestandteile der Proteine, die sich mit Aminen über eine reduktive Aminierung verknüpfen lassen.

Als Kondensationsmittel hierzu sind zahlreiche Reagenzien verfügbar, insbesondere solche, die ganz allgemein in der Organischen Synthese für Amidierungsreaktionen eingesetzt werden können (Katalog der Firma Novabiochem, 1999, Seite 264ff), wie beispielsweise Carbodiimide, insbesondere EDC (1-Ethyl-3-[3-dimethylaminopropyl]carbodiimide) Ferner werden bifunktionelle Kupplungsreagenzien mit zwei äquivalenten oder zwei orthogonalen Aktivgruppen verwendet, die entweder als Crosslinker oder zur Konjugation mit einer beliebigen weiteren Komponente (niedermolekuare Verbindung, weiteres Protein, Festphase) einsetzbar sind. Als Aktivgruppen hierfür kommen Maleinimide, Imidoester, Pyridyldisulfide, α-Halogencarbonylverbindungen und Arylazide in Frage (Katalog der Firma Pierce, 1997, Seite 133-154).

Das Labeling von Enzymen wird z.B in der Biochemie zur Detektion in Immunoassays (ELISAs), in der Immunohistochemie, beim Western Blotting und für DNA- oder RNA-Hybridisierungsassays benötigt. Insbesondere Peroxidase (Immunoassays, Immunoblots, Immunohistochemie), Alkalische Phosphatase (Immunoassays, Immunoblots), β-Galactosidase (Immunoassays, Immunoblots, Immunocytochemie mit Blutproben) und Glucoseoxidase (Immunohistochemie, Biosensorik) finden vielfältige biochemische, analytische und technische Anwendungen.

Ein typisches Beispiel für das bekannte Vorgehen ist die Herstellung von Konjugaten der Alkalischen Phosphatase (AP), indem das Reagenz Succinimidyl-4-[Nmaleimidomethyl)-cyclohexan]-1-carboxylat (SMCC) mit freien Aminogruppen der AP in einer wäßrigen Pufferlösung zur aktivierten AP umgesetzt wird. Im zweiten Schritt kann dann eine niedermolekulare Verbindung oder ein Protein, z.B. ein Antikörper, über freie Thiolgruppen an das Maleinimid der aktivierten AP verknüpft werden. Ganz analog läßt sich z.B. auch die Meerrettich-Peroxidase (HRP) an Liganden oder an Antikörper binden.

Weitere bekannte Methoden zur Herstellung von Proteinkonjugaten sind die reduktive Aminierung z.B. mit komplexen Borhydriden oder die Umsetzung mit reaktiven, kumulierten Mehrfachbindungssystemen. Zu letzteren zählen beispielsweise die Isocyanate. So werden sogenannte Neoglycoproteine beispielsweise mit BSA (Bovine Serum Albumin) hergestellt, indem bis zu 200 Äquivalente eines Isocyanat-modifizierten sialyl-Lewis-X Tetrasaccharids mit dem Protein umgesetzt werden, wobei eine Epitopendichte bis zu 16 erreicht wird (J. K. Welply, Glycobiology 4, 259-265, 1994). Weitere bekannte Methoden zur Anknüpfung von Proteinen insbesondere an feste Phasen, wie Polysaccharide, werden in US 5,059,654 beschrieben. Typische Verfahren zur kovalenten Anknüpfung von Polysacchariden an Trägerproteine mit dem Ziel der Herstellung von Vaccinen beruhen auf analogen Verfahren insbesondere unter Einsatz bifunktioneller Linker (WO 99/18121). Die für Glycoproteine und Proteoglycane geläufigen Verfahren der Modifizierung werden in folgender Referenz ausführlich beschrieben: B.Kuberan et al., Glycoconjugate J., 16, 271-281 (1999).

Die angeführten bekannten Methoden zur Herstellung von Proteinkonjugaten verwenden zumeist sehr reaktive, elektrophile Reagenzien (z.B Isocyanate, Carbodiimide), die mit den nativen, nucleophilen Gruppen des Proteins (Amin, Carboxylat, Thiol) reagieren. Für die Anbindung eines nucleophilen Antigens an ein Protein, beispielsweise zur Produktion synthetischer Vaccine, wird dessen Reaktivität durch ein geeignetes bifunktionelles Reagenz quasi "umgepolt", indem das nach reduktiver Aminierung des Oligosaccharid-Antigens gewonnene Nucleophil z.B. mit einem Succimid-Aktivester amidiert wird, der seinerseits über einen Linker mit einem elektrophilen Maleinimid verknüpft ist, welches nachfolgend über die nucleophilen Thiolgruppen des Proteins reagiert. Mit den bekannten Kupplungsreagenzien lassen sich im Prinzip auch mehrere Reportergruppen oder Liganden an das Protein hängen, sofern letztere als Mischung oder nacheinander in mehreren Verfahrensschritten umgesetzt werden. Allerdings wäre die Stöchiometrie der auf diese Weise herzustellenden Proteinkonjugate nicht kontrollierbar, da nicht alle funktionellen Gruppen des Proteins genau gleichermaßen reaktiv sind. Auch die molekulare Nähe einzuführender Liganden oder Reportergruppen im Protein wäre so keinesfalls kontrollierbar.

Andererseits wären neue Proteinkonjugate wünschenswert, in denen bis zu vier weitere funktionelle Gruppen (z.B. Liganden, Antigene) oder analytisch detektierbare Gruppen (Reportergruppen, z.B. Farbstoffe) vorliegen können. Diese Gruppen sollten alle gleichzeitig in einem einzigen einfachen Verfahrensschritt an das Protein geknüpft werden, mit exakt definierter Stöchiometrie des gebildeten Proteinkonjugates und mit strukturell definierter molekularer Distanz der funktionellen bzw. analytischen Gruppen.

Dabei können die neuen Proteine la-c auch als Mischungen vorliegen mit dem Ziel, aus einer zunächst vorhanden Proteinmischung, die z.B. aus einer Zelle oder Körperflüssigkeit gewonnen wurde, nach deren Auftrennung z.B. mittels Hochdruckflüssigkeitschromatographie (HPLC) oder zweidimensionaler Gel- oder Kapillarelektrophorese ein spezifisches, detektierbares Proteinmuster zu erzeugen, das sich vom Muster der vorhandenen Proteinmischung durch die vorgenommene Modifizierung signifikant unterscheidet.

Die oben genannten Methoden zur Herstellung von Proteinkonjugaten mit bifunktionellen Reagenzien können diese technischen Anforderungen nicht erfüllen. Entsprechendes gilt auch für spezielle höherwertige Reagenztypen: So ist z.B. das Sulfo-SBED (Pierce, 1997, Seite 151) ein trifunktionelles Molekül zur Herstellung von Proteinkonjugaten, das einen aminospezifischen Succinimidester, einen BiotinLiganden und eine unspezifische photoreaktive Gruppe (Phenylazido) in einem Reagenz vereinigt.
Aus Endeavour *18*, 115 - 122 (1994) ist weiterhin bereits bekannt, daß niedermolekulare Amine mit Carbonsäuren, Isonitrilen und einer Carbonylverbindung zu niedermolekularen Peptiden reagieren (Ugi-Reaktion). Lösliche Proteine wurden hierfür als Amino-, Carbonsäure- oder Aldehydkomponente bisher nicht verwendet. Insbesondere wurden Isonitrile als Kondensationsmittel in Verbindung mit Carbonylverbindungen hierfür bisher nicht verwendet.

### Beschreibung:

Die Erfindung betrifft neue Proteinkonjugate der in den Formeln (la-c) angegebenen Strukturen 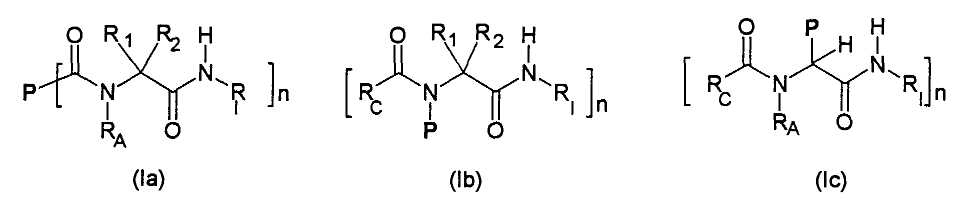 in denen **P** ein freie Amino-, Carboxyl- oder Aldehydgruppen tragendes Protein wie ein Albumin, Immunglobulin, Antikörper, Avidin, Streptavidin, Hemocyanin, Lectin, Enzym oder Serum-Glycoprotein bedeutet,
und R_{A} für einen von Aminen R_{A}-NH₂ abgeleiteten Rest steht, wobei R_{A} ein Aminogruppen tragender linearer oder verzweigter Alkyl- oder Cycloalkylrest aus bis zu 18 C-Atomen, ein Aminogruppen tragendes Alkaloid, Peptid oder Protein, Kohlenhydrat, Nucleotid, Nucleosid, Steroid, Terpen, Porphyrin, Chlorin, Corrin, Eicosanoid, Pheromon, Vitamin, insbesondere ein n-(Biotinamido)-n-alkylrest mit n = 2-8, ein Antibiotikum, Cytostatikum, ein Farbstoffmolekül oder einen Kryptanden, insbesondere Trisbipyridinium-europium(III), bedeuten,
und R_{C} für einen von Carbonsäuren R_{C}-CO₂H abgeleiteten Rest steht, wobei R_{C} ein Carboxylgruppen tragender linearer oder verzweigter Alkyl- oder Cycloalkylrest aus bis zu 18 C-Atomen, ein Carboxylgruppen tragendes Alkaloid, Peptid oder Protein, Kohlenhydrat, Nucleotid, Nucleosid, Steroid, Terpen, Porphyrin, Chlorin, Corrin, Eicosanoid, Pheromon, Vitamin, insbesondere Biotin, ein Antibiotikum, Cytostatikum, Farbstoffmolekül oder einen Kryptanden, insbesondere Trisbipyridinium-europium(lll), bedeuten,
und R_{I} für einen von Isonitrilen R_{I}-NC abgeleiteten Rest steht, wobei R_{I} ein Isonitrilgruppen tragender linearer oder verzweigter Alkyl- oder Cycloalkylrest aus bis zu 18 C-Atomen, Methoxycarbonylethyl, t-Butoxycarbonylmethyl, Phenyl, o-Alkylphenyl, m-Alkylphenyl, p-Alkylphenyl, o-Halogenphenyl, m-Halogenphenyl, p-Halogenphenyl, 2,3-Dihalogenphenyl, 2,4-Dihalogenphenyl, 3,4-Dihalogenphenyl, o-Alkoxyphenyl, m-Alkoxyphenyl, p-Alkoxyphenyl, o-Arylphenyl, m-Arylphenyl, p-Arylphenyl, o-Aryloxyphenyl, m-Aryloxyphenyl, p-Aryloxyphenyl, o-Nitrophenyl, m-Nitrophenyl, p-Nitrophenyl, 1-Naphthyl, 2-Naphthyl, Benzolsulfonylmethyl, p-Toluolsulfonylmethyl, ein Pyranosyl-, Furanosyl- oder Nucleosyl-Rest, einen n-(Biotinamido)-n-alkylrest mit n = 2-8 oder ein Farbstoffmolekül bedeuten,
und R₁ und R₂ für einen von Carbonylverbindungen abgeleiteten Rest R₁-C(=O)-R₂ stehen und gleichzeitig oder unabhängig voneinander Wasserstoff, Methyl, Ethyl, Propyl, i-Propyl, Cyclopropyl, Butyl, i-Butyl, t-Butyl, Cyclobutyl, Pentyl, Cyclopentyl, Hexyl, Cyclohexyl, 2-Methylbutyl, Heptyl, Octyl, Nonyl, Decyl, Undecyl, Dodecyl, Methoxycarbonylethyl, t-Butoxycarbonylmethyl, Phenyl, o-Alkylphenyl, m-Alkylphenyl, p-Alkylphenyl, o-Halogenphenyl, m-Halogenphenyl, p-Halogenphenyl, 2,3-Dihalogenphenyl, 2,4-Dihalogenphenyl, 3,4-Dihalogenphenyl, o-Alkoxyphenyl, m-Alkoxyphenyl, p-Alkoxyphenyl, o-Arylphenyl, m-Arylphenyl, p-Arylphenyl, o-Aryloxyphenyl, m-Aryloxyphenyl, p-Aryloxyphenyl, o-Nitrophenyl, m-Nitrophenyl, p-Nitrophenyl, 1-Naphthyl, 2-Naphthyl, Oxiranyl, Vinyl, Propenyl, Propen-2-yl, 2-Penten-2-yl, 3-Hepten-3-yl, Penta-1,3-dienyl, Phenylmethyl, 1-Phenylethyl, 2-Phenylethyl, Cyclohexen-3-yl, Cyclohexen-4-yl, 2-Furyl, 3-Furyl, 2-Thienyl, 3-Thienyl, 2-Pyrrolyl, 3-Pyrrolyl, Halogenmethyl, Dihalogenmethyl, Trihalogenmethyl, 2-Pyridyl, 3-Pyridyl, 4-Pyridyl bedeuten können
und n = 1-15, vorzugsweise 1-10 bedeutet.

Die Erfindung betrifft ferner ein Verfahren zur Herstellung der neuen Proteinkonjugate der Formeln (la-c), indem man ein lösliches Protein (**P**) oder ein an einer festen Phase immobilisiertes Protein (**P**), das freie Amino-, Carbonsäureoder Aldehyd-Gruppen trägt, mit einem Amin R_{A}-NH₂ oder einer Carbonsäuren R_{C}-CO₂H, einem Isonitril R_{I}-NC und einer Carbonylverbindung R₁-C(=O)-R₂, wobei die Reste R_{A}, R_{C}, R_{I}, R₁ und R₂ die oben angegebene Bedeutung haben, in wäßriger Lösung zu den betreffenden Protein-Konjugaten (la), (Ib) oder (Ic) umsetzt.

Dabei werden die betreffenden Strukturvarianten (la-c) - wie aus den Strukturformeln ersichtlich - aus den entsprechenden Komponenten wie folgt erhalten:
(la): aus dem Protein (**P**), dem Amin R_{A}-NH₂, dem Isonitril R_{I}-NC und der Carbonylverbindung R₁ -C(=O)-R₂,
(Ib): aus dem Protein (**P**), der Carbonsäure R_{C}-CO₂H, dem Isonitril R_{I}-NC und der Carbonylverbindung R₁ -C(=O)-R₂,
(Ic): aus dem oxidierten Protein (**P**), der Carbonsäure R_{C}-CO₂H, dem Amin R_{A}-NH₂ und dem Isonitril R_{I}-NC.

Das erfindungsgemäße Verfahren ist besonders geeignet für die Umsetzung von Proteinen (**P**), die freie Aminogruppen oder freie Carboxylatgruppen oder freie Aldehydgruppen tragen. Insbesondere geeignet sind die folgenden Proteine entweder in freier oder festphasengebundener Form:
- Trägerproteine, die mit Haptenen (Peptiden oder Kohlenhydrate) als immunogene Epitope verknüpft werden sollen, z.B. KLH (Keyhole Limpet Hemocyanin), CRM (nicht toxische Diphtherie-Toxinvarianten) oder Albumine wie Rinderserum-Albumin (BSA: Bovine Serum Albumin).
- In der Immunologie verwendete native oder rekombinante Proteine mikrobiellen Ursprungs, z.B. die Proteine A, G oder A/G, die den Fc-Teil von Immunglobulinen, z.B. in IgG, erkennen. Ferner Avidin und Streptavidin sowie Lectine wie z.B. Concanavalin A oder Weizenkeim-Agglutinin (WGA) und Peptidoglycane
- Immunglobuline IgG, IgM, IgA, auch in oxidierter Form, z.B. oxidiertes IgG.
- Enzyme, wie z.B. Pepsin, Papain, Trypsin, Ficin, Chymotrypsin, Lipasen, Esterasen, Oxidoreduktasen, Transaminasen, Glycosidasen, z.B. β-Galactosidase, Glycosyltransferasen, Amidasen, Hydantoinasen, Meerrettich-Peroxidase (HRP: Horse Radish Peroxidase, Alkalische Phosphatase (AP).

Die Umsetzung des Proteins (**P**) mit dem Amin R_{A}-NH₂ oder der Carbonsäure R_{C}-CO₂H, und dem Isonitril R_{I}-NC und der Carbonylverbindung R₁-C(=O)-R₂ erfolgt beim erfindungsgemäßen Verfahren derart, daß zu einer Lösung oder einer Suspension von (**P**) in wäßrigem Puffer, der die weiteren Komponenten jeweils in mindestens molarer Menge zum Protein enthält, nacheinander das Amin R_{A}-NH₂ oder die Carbonsäure R_{C}-CO₂H und das Isonitril R_{I}-NC hinzu gegeben werden. Hierbei werden die jeweils benötigten Komponenten Amin R_{A}-NH₂, Carbonsäure R_{C}-CO₂H, Isonitril R_{I}-NC und die Carbonylverbindung R₁-C(=O)-R₂ in einem 10-10.000 fachen molaren Überschuß zum Protein (**P**) eingesetzt, vorzugsweise im molaren Verhältnis 100-1.000, besonders bevorzugt im Verhältnis 200-1000.

Als Reaktionslösung eignen sich wäßrige Puffer wie beispielsweise 0.001 - 1.0 molare Lösungen von Natrium- oder Kalium-dihydrogenphosphat mit Dinatrium- oder Dikalium-hydrogenphosphat oder-Tris(hydroxymethyl)-aminomethan mit Salzsäure, bevorzugt geeignet sind Pufferlösungen für den pH-Bereich zwischen 5 und 9, besonders bevorzugt zwischen pH 6 und pH 8.

Dem Puffer können bei dem erfindungsgemäßen Verfahren die Cosolventien Methanol, Ethanol, Propanol, i-Propanol, Butanol, Essigsäure-ethylester, Essigsäuremethylester, Dimethylformamid, Acetonitril, Dimethylsulfoxid oder Sulfolan in Mengen von 0.1-20 Vol-% zugesetzt werden, je nach den Löslichkeitserfordernissen der Reaktionspartner. Die Reaktionstemperatur liegt zwischen 0°C und 90°C, bevorzugt bei 10°C bis 40°C, besonders bevorzugt bei Raumtemperatur.

Die durch das erfindungsgemäße Verfahren erhältlichen rohen Proteinkonjugate (la-c) können weiterhin durch Dialyse mit wäßrigen Pufferlösungen oder reinem Wasser sowie durch dem Fachmann geläufige biochemische Chromatographie-Verfahren gereinigt und dann der weiteren Anwendung zugeführt werden.

Der exakte Struktumachweis der Produkte (la-c), das heißt die analytische Bestimmung der mittleren Anzahl (n) der am Protein (**P**) gebundenen Komponenten, erfolgt durch die direkte Messung des Molekulargewichtes mittels MALDI-TOF-Massenspektrometrie oder durch die selektive Bestimmung einer oder mehrerer an das Protein gebundener Komponenten. So läßt sich die beispielsweise die Menge eines über die Komponenten R_{A}-NH₂, R_{C}-CO₂H oder R_{I}-NC eingeführten Farbstoffmoleküls in einfacher Weise über die UV-Extinktion messen. Ein analog eingeführtes Nucleotid läßt sich - gegebenenfalls nach Amplifikation mittels der PCR-Verfahren - nach den geläufigen biochemischen Verfahren quantifizieren.

Die resultierende berechnete Zahl (n) beschreibt die Stöchiometrie, d.h die sogenannte mittlere Epitopdichte am Protein (**P**) der Formeln (la-c).

So kann die praktische Durchführung des erfindungsgemäßen Verfahrens beispielsweise so erfolgen, daß zuerst ein Protein (**P**), wie z.B. das Rinderserumalbumin, bei Raumtemperatur (ca. 20°C) in 0.1 molarem Phosphatpuffer (pH-Wert 7.5), der die Carbonylverbindung R₁-C(=O)-R₂ im molaren Verhältnis zwischen 100 - 4.000 enthält, gelöst wird. Anschließend wird zu dieser Lösung nacheinander R_{A}-NH₂ oder R_{C}-CO₂H sowie R_{I}-NC im entsprechenden molaren Verhältnis zwischen 100 - 4.000 (bezogen auf **P**) gegeben. Nach zwei Tagen Reaktionszeit bei Raumtemperatur werden die Überschüsse der niedermolekularen Reaktionskomponenten durch Dialyse mit Puffer oder reinem Wasser oder durch Chromatographie entfernt. Die Lösung oder Suspension des so erhältlichen neuen Proteinkonjugates wird dann wie oben beschrieben analytisch charakterisiert.

Durch Variation der insgesamt 4 Reaktionskomponenten 1. Amin R_{A}-NH₂, 2. Carbonylverbindung R₁-C(=O)-R₂, 3. Carbonsäure R_{C}-CO₂H und 4. Isonitril R_{I}-NC - wobei das Protein (**P**) je nach Auswahl der verbleibenden 3 im Überschuß eingesetzten Reaktionskomponenten entweder als Amin R_{A}-NH₂, Carbonylverbindung R₁-C(=O)-R₂ oder als Carbonsäure R_{C}-CO₂H eingesetzt wird - ergeben sich mannigfaltige Möglichkeiten der Anbindung nahezu beliebiger Komponenten an (**P**), wobei über Variation der betreffenden Reste R bis zu 4 verschiedene Typen von Liganden (z.B. Biotin) und/oder Reportergruppen (z.B. Farbstoffe) in exakt definiertem stöchiometrischem Verhältnis 1:1:1:1 und in definiertem molekularem Abstand relativ zueinander und relativ zum Protein eingeführt werden. Die molekularen Verhältnisse lassen sich hierbei noch durch die Auswahl der Spacerlängen in den Resten R_{A}, R₁, R₂, R_{C} und R_{I} einstellen.

Eine kleine Auswahl der sich daraus ergebenden vielfältigen Möglichkeiten beispielsweise zur Konjugation von 2 verschiedenen Farbstoffen (F1 und F2) sowie eines weiteren beliebigen Liganden, z. B. von Biotin (B) wird durch folgende Formeln illustriert:

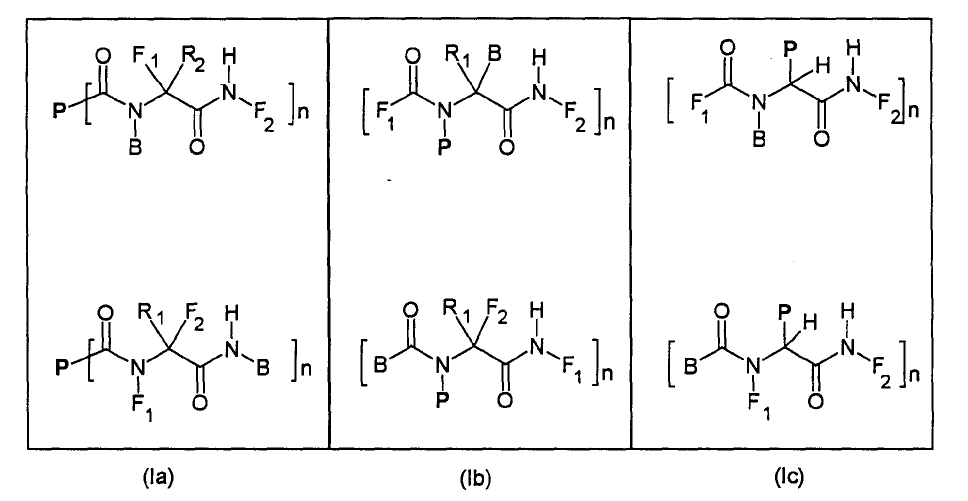

Ein Beispiel ist z.B. die Verwendung eines Farbstofflabels als Carboxylkomponente R_{C}-CO₂H und eines Biotinliganden als Aminkomponente R_{A}-NH₂:

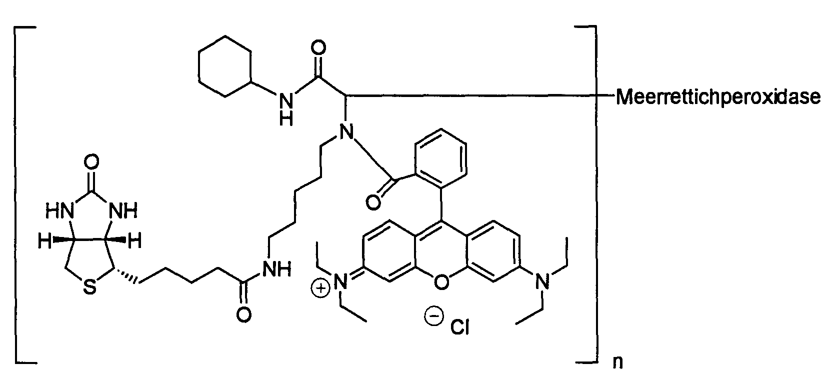

Diese Beispiel illustriert das Anwendungspotential der erfindungsgemäßen Proteinkonjugate und die Einfachheit des Verfahrens ihrer Herstellung: Die vier Reaktionskomponenten
1. Oxidierte Protein Meerrettichperoxidase als Carbonylkomponente HC(=O)-R₂,
2. Rhodamin Farbstofflabel als Carboxylkomponente R_{C}-CO₂H
3. Biotinliganden als Aminkomponente R_{A}-NH₂
4. Cyclohexylisonitril als R_{I}-NC
werden einfach im gewünschten stöchimetrischen Verhältnis vereinigt und unter milden Bedingungen im wäßrigen Puffer umgesetzt. Der skizzierte Reaktionsverlauf und die Struktur des neuen Proteinkonjugates läßt sich nun exakt anhand unabhängiger funktionaler Bestimmungsverfahren für die beiden Reportergruppen (Biotin, Farbstoff) charakterisieren: So ergibt die Molmassenbestimmung mittels MALDI-TOF eine mittlere Epitopendichte n = 3.4, die durch einen Biotin-ELISA-Test (n = 3.3) und die UV-Extinktionsmessung (n = 3.5) abgesichert wird. Die Aktivität des Enzyms (Restaktivität 93%) bleibt im Enzymassay weitgehend erhalten.

Umgekehrt könnte auch Rhodamin B über einen geeigneten Linker als Aminkomponente R_{A}-NH₂ und Biotin als Carboxylkomponente R_{C}-CO₂H eingesetzt werden, um nur eine der vielfältig möglichen Variationen aufzuführen. Die stöchiometrisch vorliegende Reportergruppe kann also zur direkten internen Bestimmung einer weiteren Reportergruppe herangezogen werden, und umgekehrt, was die Genauigkeit der Bestimmungen erhöht. So kann im oben aufgeführten Beispiel der Farbstoff zur direkten internen Bestimmung des Biotinylierungsgrades dienen, und umgekehrt. Dies kann wesentlich einfacher sein als die geläufigen Verfahren zur Biotinbestimmung in Proteinen, bei denen indirekte Methoden eingesetzt werden: So verwendet z.B. die HABA-Avidin-Methode (HABA = 2-Hydroxyazobenzol-4'-carbonsäure) die Bindung an Avidin unter Bildung eines farbigen Komplexes (Absorption 500 nm), der durch freies Biotin verdrängt wird. Aus mehreren Meßpunkten wird eine Standardkurve erstellt, aus der Biotinylierugsgrad bestimmt wird.

Die erfindungsgemäßen Proteinkonjugate (la-c) lassen sich also vielfältig beispielsweise auch mit dem System des Biotin-Avidin-Komplexes (ABC) zur Detektion und Quantifizierung von Antigenen, z.B. an festen Phasen, verwenden. Dazu gehören naturgemäß auch sämtliche auf diesem System beruhende biochemische Assaymethoden, wie z.B. der klassische ELISA-Assay, Fluoreszenzaktivierte Zellsortierung (FACS), Affinitätschromatographie und das Western Blotting. Avidin, Streptavidin, Meerrettichperoxidase, Glucoseoxidase und Alkalische Phosphatase sind häufig verwendete Proteine und Enzyme in diesen Systemen. Häufig verwendete Farbstoffe sind z.B. Fluorescein, (FITC), Rhodamin B und Texasrot, aber auch Varianten dieser Moleküle mit verbesserten Fluoreszenzeigenschaften können im Sinne des erfindungsgemäßen Verfahrens verwendet werden, beispielsweise Konjugate von Sulforhodaminen (US 5,846,737).

Die erfindungsgemäßen Proteinkonjugate (la-c) lassen sich demnach vielfältig in die immunologischen Methoden der klinischen Diagnostik integrieren, mit deren Hilfe beispielsweise Plasmaspiegel von Analyten im Blut kontrolliert werden, hormonelle Regelkreise überwacht werden (Schilddrüsenfunktion, Fertilität), virale Infektionen nachgewiesen werden (Hepatitis, HIV) oder Tumor-assoziierte Proteine detektiert werden (Tumornachsorge). Makromoleküle wie Peptide, Proteine und Nucleinsäuren werden in der Regel über heterogene Immunoassays (Festphase z.B. als Träger eines Antikörpers) bestimmt, während für niedermolekulare Substanzen auch homogene Methoden wie der Fluoreszenz-Polarisationsassay (FPIA) oder Enzymemultiplied Immunoassay (EMIT) verwendet werden. Neuere Methoden haben eine gesteigerte Sensitivität, indem der Analyt (z.B. ein Tumormarkerprotein) gleichzeitig an einen monoklonalen Antikörper, konjugiert mit einem Europium(III)-Komplex, und an einen weiteren Antikörper, gekoppelt an ein Farbstoffmolekül (Allophycocyanin) bindet. Nach der Theorie von *Förster* führt die eingestrahlte monochromatische Lichtenergie bei der Bindung durch die molekulare räumliche Nähe von Lanthanidkomplex und Farbstoffmolekül zu einem strahlungslosen Übergang von ersterem auf letzteres, und dadurch wird eine langlebige und amplifizierte Emission hervorgerufen (Time-resolved Amplified Cryptate Emission Asssay, TRACE) (Bioforum, *1*-2, 1998, 14-16). In ähnlicher Weise kann die räumliche Nähe von einen oder mehreren Farbstoffen (multiple chromophore Donorgruppen) mit einem Fluoreszenz-Akzeptorfarbstoff zur Signalamplifikation genutzt werden (US 5,849,489). Eine besonders empfindliche Spurenanalyse von Biomolekülen beruht auf der sogenannten Fluoreszenz-Cross-Corelation (FCS) -Spektroskopie, bei der die Lichtverstärkung durch zwei fluorogene Farbstoffe in definierter räumlicher Nähe erfolgt. Die Einstrahlung mit zwei entsprechenden Laserquellen führt zu Energietransfers und einer verstärkten Fluoreszenz-Emission des markierten Moleküls, die mit Photonen-Detektoren in konfokaler Anordnung gemessen wird. Verwendet werden z.B. die Farbstoffe Rhodamin-Grün und Cy5 (beide von Amersham Life Sciences erhältlich). Die Grundlagen dieser Methodik, mit deren Hilfe wenige Kopien einer amplifizierten DNA quantifiziert wurden, finden sich beschrieben in: R.Rigler et al., J. Biotechnol., 63, 97-109 (1998).

Da das erfindungsgemäße Verfahren die Anknüpfung von bis zu 4 Reportgruppen in definierter Stöchiometrie und einstellbarer molekularer Distanz erlaubt, lassen entsprechende Proteinkonjugate (la-c) mit diesen Farbstofftypen (Beispiele für entsprechende Derivate in US 5,849,489) ebenfalls eine stark gesteigerte Empfindlichkeit der Detektion des Proteins selbst bzw. eines gebundenen Liganden bzw. eines im Immunoassay bestimmten zu bestimmenden Analyten erwarten.

Das erfindungsgemäße Verfahren eignet sich insbesondere auch zur gezielten Konjugation von Sacchariden mit definierter Epitopdichte am Protein (siehe Beispiele). Letztere kann z.B. über die stöchiometrische, simultane Anbindung eines Farbstoff-Reportermoleküls in einer der Reaktionskomponenten bestimmt werden. Dies eröffnet die Möglichkeit zur kontrollierten Anbindung von mehreren Saccharid-Antigenen, beispielsweise von verschiedenen bakteriellen oder viralen Serotypen (z.B. Influenza, Hepatitis) zu noch höhervalenten Vaccinen oder von mehreren Tumor-assoziierten Antigenen für Tumorvaccine, beispielsweise an hierfür geeignete Proteine wie CRM (nicht-toxische Diphterietoxin-Variante) oder an das KLH-Protein

Das erfindungsgemäße Verfahren eignet sich ferner zur kovalenten Anknüpfung von einem oder von bis zu drei verschiedenen Cytostatika an ein Protein, beispielsweise an einen Tumor-spezifischen Antikörper oder an ein Enzym, z.B. an eine Glycosidase, oder an ein Glycoprotein, das von einem zellspezifischen Lectin erkannt wird, wie z.B. an Asialoglycoproteine, die von Leberzellen-Lectinen erkannt werden.

Das erfindungsgemäße Verfahren eignet sich ferner zur Umsetzung von Proteinmischungen mit dem Ziel, ein spezifisch detektierbares Proteinmuster nach entsprechender Auftrennung dieser Proteinmischung zwecks analytischer Charakterisierung des Proteoms eines Organismus (Proteomics). Hierbei wird die Probenvorbereitung lediglich um den erfindungsgemäßen Verfahrensschritt ergänzt, bevor das übliche zweidimensionale Elektrophorese-Chromatogramm (das sogenannte 2 DE map) angefertigt wird. Übliche Methoden zur Visualisierung der Proteinspots auf Gelen als stationärer Phase arbeiten mit Chromassie Blue oder Silbernitrat (Staining) sowie mit Fluoreszenzfarbstoffen. Die Auftrennung in zwei Dimensionen erfolgt nach den Molmassen und nach den isoelektrischen Punkten (pl-Werte) der Proteine, wobei basische Proteine z.B. bis pl 9 und saure Proteine bis pl 4-5 detektiert werden.

Jenseits dieser pl-Werte liegende Proteine werden schlechter oder gar nicht getrennt bzw. detektiert. Hier bietet das erfindungsgemäße Verfahren eine technische Lösung zur besseren Auftrennung und Detektion, indem stark basische Proteine als Aminkomponente mit einer niedermolekularen Carbonsäure R_{C}-CO₂H, einem Isonitril R_{I}-NC und einer Carbonylverbindung R₁-C(=O)-R₂ umgesetzt werden, wobei mindestens eine der letzteren drei Komponenten z.B. jeweils Farbstoffe oder Biotinlabel enthalten. Stark saure Proteine werden sinngemäß als Carboxylkomponente mit dem Amin R_{A}-NH₂ und dem Isonitril R_{I}-NC und der Carbonylverbindung R₁-C(=O)-R₂umgesetzt. Glycoproteine werden in der oxidierten Form als Carbonylkomponente mit dem Amin R_{A}-NH₂, der Carbonsäure R_{C}-CO₂H und dem Isonitril R_{I}-NC umgesetzt. Es ergeben sich also drei neue Möglichkeiten der Mustererzeugung von Proteinmischungen, wobei die neu entstandene Gruppierungen von Proteinen durch das anhängende Label identifiziert werden können. Bei konsekutiver Anwendung aller Modifizierungen könnten entsprechende Gruppen von Proteinen gleichzeitig anhand ihrer unterschiedlichen Label zugeordnet werden.

Die Erfindung wird durch die nachfolgenden Ausführungsbeispiele näher erläutert.

### Beispiel 1

### Herstellung von (la) für P = Rinderserumalbumin (BSA), R₁, R₂ = CH₃, R_{I} = Cyclohexyl, R_{A} = 5-(β-D-Glucopyranosyloxy)pentyl, n = 7.7.

Zu 3 ml 0.1-molarem Phosphatpuffer, pH 7.5, gibt man bei Raumtemperatur eine Lösung von Rinderserumalbumin (1 mg, 0.015 µmol) in Wasser (1 ml), 5-%ige Acetonlösung in Wasser (67.2 µl, 45 µmol), 5-Aminopentyl-β-D-glucopyranosid (13.59 mg, 45 µmol) und 2 Tropfen Cyclohexylisonitril. Die Lösung wird 2 Tage bei Raumtemperatur stehen gelassen und mit einer Ultrafiltrationszelle konzentriert. Das rohe Proteinkonjugat wird anschließend einmal gegen 0.1 molaren Phosphatpuffer, pH 7.5, und zweimal gegen reines Wasser dialysiert. Die Molmasse Produktes wird mittels MALDI-TOF-Massenspektrometrie bestimmt: Molekulargewicht: 69978 Da, d.h. eine mittlere Epitopendichte von n=7.7.

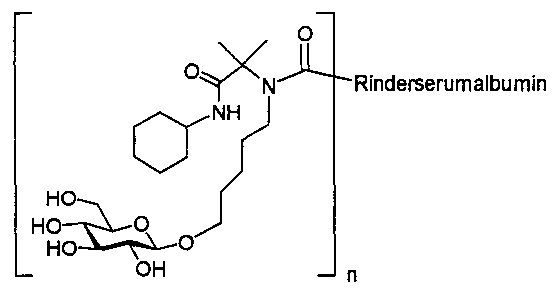

### Beispiel 2

### Herstellung von (la) für P = Rinderserumalbumin (BSA), R₁, R₂ = CH₃, R_{I} = Cyclohexyl, R_{A} = 5-(β-D-Glucopyranosyloxy)pentyl, n = 1.0.

Analog Beispiel 1 werden in 0,1-molarem Trispuffer, pH 7.5, Rinderserumalbumin (1 mg, 0.015 µmol), 5-%ige Acetonlösung in Wasser (2.24 µl, 1,5 µmol), 5-Aminopentyl-β-D-glucopyranosid (0.45 mg, 1.5 µmol) und 1 Tropfen Cyclohexylisonitril umgesetzt. Ergebnis: Molekulargewicht: 66898 Da, mittlere Epitopendichte: n=1.0; Formel siehe Beispiel 1.

### Beispiel 3

### Herstellung von (la) für P = Rinderserumalbumin (BSA), R₁, R₂ = CH₃, R_{I} = Methoxycarbonylmethyl, R_{A} = 5-(β-D-Glucopyranosyloxy)pentyl, n = 8.8.

Analog Beispiel 1 werden in 0.1-molarem Phosphatpuffer, pH 7.5, Rinderserumalbumin (1 mg, 0.015 µmol), 5-%ige Acetonlösung in Wasser (22.4 µl, 15 µmol), 5-Aminopentyl-β-D-glucopyranosid (4.5 mg, 15 µmol) und 1 Tropfen Isocyanessigsäure-methylester umgesetzt. Ergebnis: Molekulargewicht: 67948 Da, d.h. mittlere Epitopendichte: n=3.4

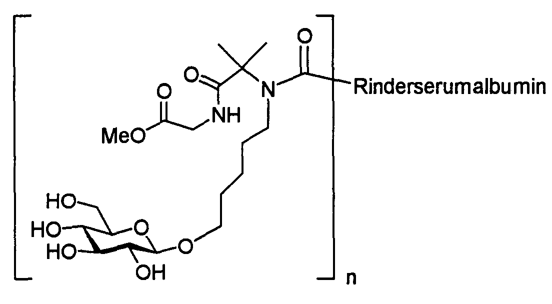

### Beispiel 4

### Herstellung von (la) für P = Rinderserumalbumin (BSA), R₁, R₂ = CH₃, R_{I} = Cyclohexyl, R_{A} = 5-(2-Acetamido-2-desoxy-β-D-glucopyranosyloxy)pentyl, n = 8.8.

Analog Beispiel 1 werden in 0.1-molarem Phosphatpuffer, pH 7.5, Rinderserumalbumin (1 mg, 0.015 µmol), 5-%ige Acetonlösung in Wasser (44.8 µl, 30 µmol), 5-Aminopentyl-2-acetamido-2-desoxy-β-D-glucopyranosid (10.28 mg, 30 µmol) und 2 Tropfen Cyclohexylisonitril umgesetzt. Ergebnis: Molekulargewicht: 70480 Da, d.h. mittlere Epitopendichte: n=8.8.

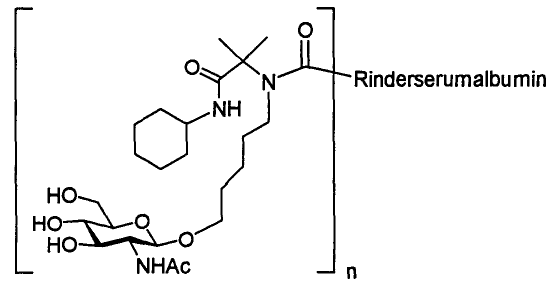

### Beispiel 5

### Herstellung von (Ib) für P = Rinderserumalbumin (BSA), R₁, R₂ = CH₃, R_{I} = Cyclohexyl, R_{C} = (2-O-Acetyl-β-D-glucopyranosyloxy)methyl, n = 9.9.

Analog Beispiel 1 werden in 0.1-molarem Phosphatpuffer, pH 7.5, Rinderserumalbumin (1 mg, 0.015 µmol), 5-%ige Acetonlösung in Wasser (89.6 µl, 60 µmol), Carboxymethyl-2-O-acetyl-β-D-glucopyranosid (19.1 mg, 60 µmol) und 2 Tropfen Cyclohexylisonitril umgesetzt. Molekulargewicht: 70975 Da, mittlere Epitopendichte: n=9.9

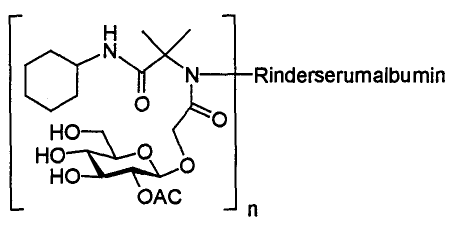

### Beispiel 6

### Herstellung von (Ib) für P = Rinderserumalbumin (BSA), R₁, R₂ = CH₃, R_{I} = Cyclohexyl, R_{C} = Rhodamin B, n = 4.1-4.8.

Analog Beispiel 1 werden in 0.1-molarem Phosphatpuffer, pH 7.0, (3932,8 µl), Rinderserumalbumin (1 mg, 0.015 µmol), 5-%ige Acetonlösung in Wasser (67.2 µl, 45 µmol), Rhodamin B (21.6 mg, 45 µmol, in 400 µl Wasser) und 2 Tropfen Cyclohexylisonitril umgesetzt. Die Bestimmung der Konjugatausbeute und der mittleren Epitopdichte erfolgt photometrisch durch Messung der Extinktionen bei 280 nm (Rinderserumalbumin) und 575 nm (Rhodamin B).

| Ergebnis nach 1 Tag Reaktionszeit: | |
|---|---|
| Rinderserumalbumin: 7.4 nmol | Rhodamin B: 30.4 nmol |
| Konjugatausbeute: 49% | mittlere Epitopdichte: n=4.1 |

| Ergebnis nach 2 Tagen Reaktionszeit: | |
|---|---|
| Rinderserumalbumin: 1,2 nmol | Rhodamin B: 6,0 nmol |
| Konjugatausbeute: 8.3% | mittlere Epitopdichte: n=4.8 |

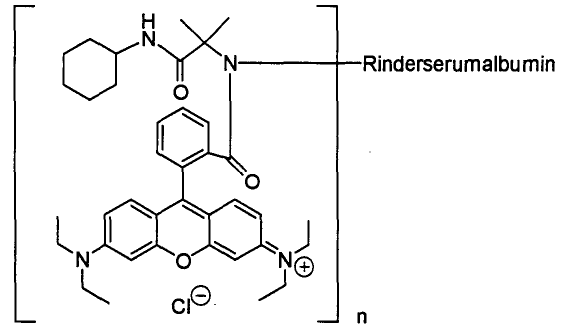

### Beispiel 7

### Herstellung von (Ib) für P = Rinderserumalbumin (BSA), R₁, R₂ = CH₃, R_{I} = 1β-D-Glucopyranosyl, R_{C} = Rhodamin B, n = 6.8-12.5.

Analog Beispiel 1 werden in 0.1-molarem Phosphatpuffer, pH 7.0 (2932,8 µl), Rinderserumalbumin (1 mg, 0.015 µmol), 5-%ige Acetonlösung in Wasser (67.2 µl, 45 µmol), Rhodamin B (400 µg, 0.835 µmol, in 400 µl Wasser) und β-D-Glucopyranosylisonitril (1.9 mg, 10 µmol, in 1 ml Wasser) umgesetzt. Das β-D-Glucopyranosylisonitril wird frisch aus 2,3,4,6-Tetra-O-acetyl-β-D-glucopyranosylisonitril (3.6 mg, 10 µmol) durch Lösen in 5 ml Methanol, 5 h Rühren bei RT mit einer katalytischen Menge NaOMe, Neutralisieren mit Dowex H+ lonentauscher, Filtrieren und Konzentrieren des Filtrats hergestellt. Die Bestimmung der Konjugatausbeute und der mittleren Epitopdichte erfolgt photometrisch durch Messung der Extinktionen bei 280 nm (Rinderserumalbumin) und 575 nm (Rhodamin B).

| Ergebnis nach 2 Tagen Reaktionszeit: | |
|---|---|
| Rinderserumalbumin: 15 nmol | Rhodamin B: 6,8 nmol |
| Konjugatausbeute: 100% | mittlere Epitopdichte: n=0.45 |

| Ergebnis nach 4 Tagen Reaktionszeit: | |
|---|---|
| Rinderserumalbumin: 12 nmol | Rhodamin B: 12,5 nmol |
| Konjugatausbeute: 80% | mittlere Epitopdichte: n=1.04 |

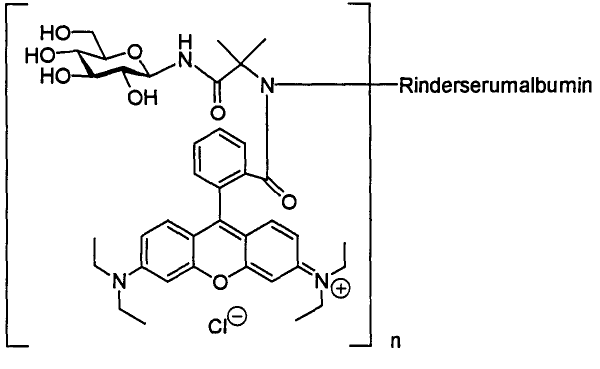

### Beispiel 8

### Herstellung von (Ib) für P = Meerrettichperoxidase (HRP), R₁ = H, R₂ = CH₂CH₃, R_{I} = Cyclohexyl, R_{C} = 5-(Biotinoylamino)pentyl, n = 3.0

Analog Beispiel 1 werden in 0.1-molarem Phosphatpuffer, pH 7.5 (4000 µl), Meerrettichperoxidase (1.9 mg, 0.049 µmol HRP), (+)-Biotinaminocapronsäure (14.5 mg, 45 µmol), Propionaldehyd (2.4 µg, 0.05 µmol) und Cyclohexylisonitril (2 Tropfen) umgesetzt. Die Bestimmung der Konjugatausbeute erfolgt photometrisch durch Messung der Extinktion bei 280 nm. Die Restaktivität des Peroxidasekonjugats erfolgt photometrisch im Vergleich zu nativer Peroxidase durch Oxidation einer ABTS-Farbstofflösung und Messung der Extinktion bei 404 nm. Die quantitative Bestimmung des Biotinylierungsgrads erfolgt photometrisch durch Verdrängung von HRP-BA aus seinem Avidinkomplex und Messung der Extinktion bei 500 nm sowie qualitativ durch ELISA-Test an Streptavidin-belegten Mikrotiterplatten.

| Ergebnis nach 1 Tag Reaktionszeit: | |
|---|---|
| Peroxidasekonjugat: 36 nmol (75%) | Restaktivität: 62% |
| Biotingehalt: 108 nmol | Epitopdichte: n=3.0 |

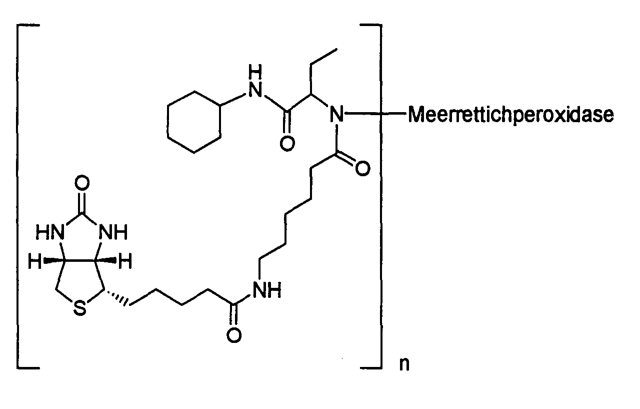

### Beispiel 9

### Herstellung von (Ic) für P = ox-Meerrettichperoxidase (ox-HRP), R_{I} = Cyclohexyl, R_{C} = Rhodamin B, R_{A} = = 5-(Biotinoylamino)pentyl, n = 3.3-3.4

Analog Beispiel 1 werden in 0.1-molarem Phosphatpuffer, pH 7.5 (4000 µl), NalO₄oxidierte Meerrettichperoxidase (1.8 mg, 0.045 µmol), (+)-Biotinamidopentylamin (14.8 mg, 45 µmol), Rhodamin B (21.6 mg, 45 µmol) und Cyclohexylisonitril (2 Tropfen) umgesetzt. Die Bestimmung der Konjugatausbeute erfolgt photometrisch durch Messung der Extinktion bei 280 nm (Peroxidasekonjugat) und 575 nm (Rhodamin B). Die Bestimmung der Restaktivität des Peroxidasekonjugats erfolgt photometrisch im Vergleich zu nativer Peroxidase durch Oxidation von ABTS-Lösung und Messung der Extinktion bei 404 nm. Die quantitative Bestimmung des Biotinylierungsgrads erfolgt photometrisch durch Verdrängung von ox-HRP-BA aus seinem Avidinkomplex und Messung der Extinktion bei 500 nm, sowie qualitativ durch einen ELISA-Test an Streptavidin-belegten Mikrotiterplatten.

| Ergebnis nach 1 Tag Reaktionszeit: | |
|---|---|
| Molekulargewicht: 46118 Da | Epitopendichte: n = 3.4 |
| Peroxidasekonjugat: 4.8 nmol (11%) | Restaktivität: 93% |
| Rhodamin B-Gehalt: 16.8 nmol | Epitopendichte: n=3.5 |
| Biotingehalt: 16 nmol | Epitopdichte: n=3.3 |

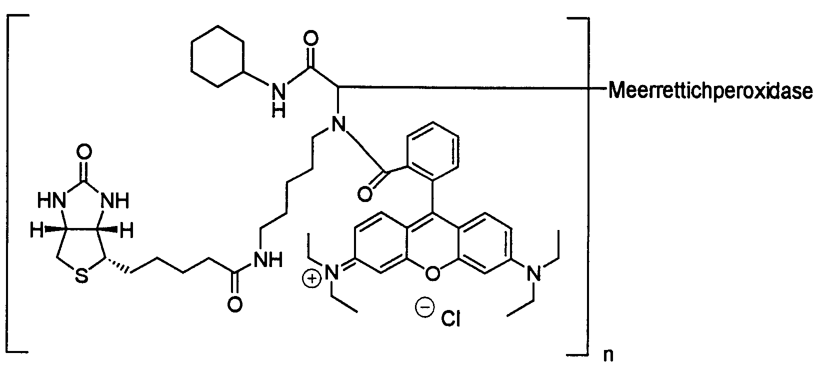

### Beispiel 10

### Herstellung von (Ic) für P = ox-Meerrettichperoxidase (ox-HRP), R_{I} = Cyclohexyl, R_{C} = 5-(Biotinoylamino)pentyl, R_{A} = = 5-(Biotinoylamino)pentyl, n = 1.0

Analog Beispiel 1 werden in 0.1-molarem Phosphatpuffer, pH 7,5 (4000 µl), NalO₄oxidierte Meerrettichperoxidase (1 mg, 0.026 µmol), (+)-Biotinamidopentylamin (14.8 mg, 45 µmol), (+)-Biotinaminocapronsäure (14.5 mg, 45 µmol) und Cyclohexylisonitril (2 Tropfen) umgesetzt. Die Bestimmung der Konjugatausbeute erfolgt photometrisch durch Messung der Extinktion bei 280 nm. Die Messung der Restaktivität des Peroxidasekonjugats erfolgt photometrisch im Vergleich zu nativer Peroxidase durch Oxidation von ABTS-Lösung und Messung der Extinktion bei 404 nm. Die quantitative Bestimmung des Biotinylierungsgrads erfolgt photometrisch durch Verdrängung von ox-HRP-BA aus seinem Avidinkomplex und Messung der Extinktion bei 500 nm sowie qualitativ durch ELISA-Test an Streptavidin-belegten Mikrotiterplatten.

| Ergebnis nach 1 Tag Reaktionszeit: | |
|---|---|
| Peroxidasekonjugat: 16 nmol (67%) | Restaktivität: 157% |
| Biotingehalt: 16 nmol | Epitopdichte: n=1.0 |

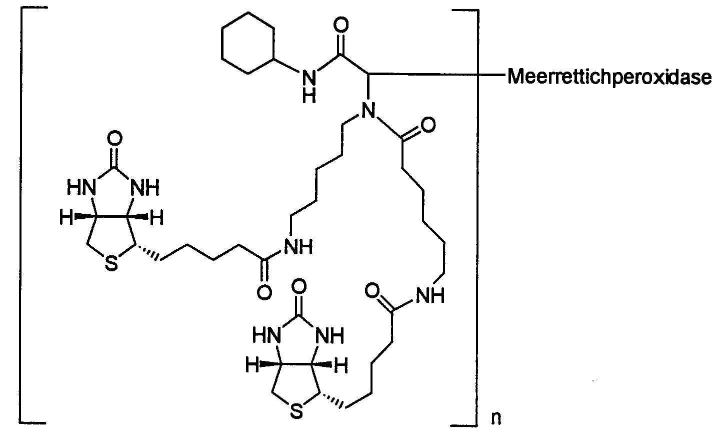

### Beispiel 11

Proteomanalyse: Die löslichen Proteine eines Mikroorganismus, z.B. aus den Zellen einer Hefe oder eines *E.coli* Bakteriums, oder eines Säugerorganismus, z.B. aus dem Blutserum eines Menschen, werden nach den geläufigen Verfahren gewonnen (Zellaufschluß, Extraktion, Waschen in Puffern etc.). Anstelle einer direkten Auftragung auf ein Elektrophorese-Gel wird vorher die Derivatisierung der Proteinmischung in wäßrigem Puffer - in ganz analoger Weise wie in einem der vorstehenden Beispiele beschrieben - durchgeführt. Nach zweidimensionaler Auftrennung wird das Gel wie üblich z.B. mit Cromassie Blue, mit Silbernitrat oder mit einem Fluoreszenz-Farbstoff entwickelt. War zumindest eine der drei eingesetzten niedermolekularen Reaktionskomponenten bereits ein Derivat eines geeigneten Fluoreszenzfarbstoffes, so lassen sich die entsprechend modifizierten Proteine direkt mit einem Lesegerät nachweisen. Das Image-Prozessing (z.B. mittels Programm Melanie II, PC-NT der Firma Biorad) und das sich anschließende Rechner-gestützte Prozessing (d.h. die Mustererkennung und -auswertung) werden wie bei der Auswertung zweidimensionalen Elektrophorese-Chromatogramme üblich vorgenommen. Zusätzlich können die interessierenden Gel-Spots wie üblich mit einem Gel-Picker aufgenommen werden und nach Trypsin-Verdauung der massenspektrometrischen Analyse zugeführt werden (MALDI-TOF, ESI, MS-MS).

## Patentansprüche

1. Proteinkonjugate der in den Formeln (la-c) angegebenen Struktur, 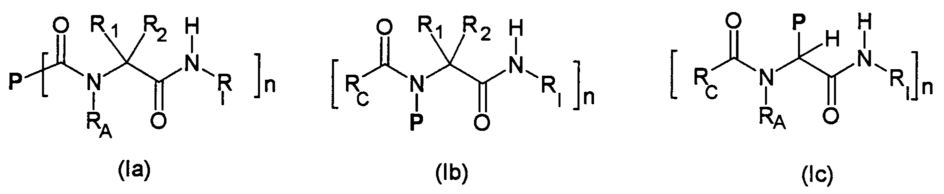 wobei **P ein freie Amino-, Carboxyl- oder Aldehydgruppen tragendes Protein wie ein** Albumin, Immunglobulin, Antikörper, Avidin, Streptavidin, Hemocyanin, Lectin, Enzym oder Serum-Glycoprotein bedeutet,
und R_{A} für einen von Aminen R_{A}-NH₂ abgeleiteten Rest steht, wobei R_{A} ein Aminogruppen tragender linearer oder verzweigter Alkyl- oder Cycloalkylrest aus bis zu 18 C-Atomen, ein Aminogruppen tragendes Alkaloid, Peptid oder Protein, Kohlenhydrat, Nucleotid, Nucleosid, Steroid, Terpen, Porphyrin, Chlorin, Corrin, Eicosanoid, Pheromon, Vitamin, insbesondere ein n-(Biotinamido)-n-alkylrest mit n = 2-8, ein Antibiotikum, Cytostatikum, ein Farbstoffmolekül oder einen Kryptanden, bedeuten,
und R_{C} für einen von Carbonsäuren R_{C}-CO₂H abgeleiteten Rest steht, wobei R_{C} ein Carboxylgruppen tragender linearer oder verzweigter Alkyl- oder Cycloalkylrest aus bis zu 18 C-Atomen, ein Carboxylgruppen tragendes Alkaloid, Peptid oder Protein, Kohlenhydrat, Nucleotid, Nucleosid, Steroid, Terpen, Porphyrin, Chlorin, Corrin, Eicosanoid, Pheromon, Vitamin, insbesondere Biotin, ein Antibiotikum, Cytostatikum, ein Farbstoffmolekül oder einen Kryptanden bedeuten,
und R_{I} für einen von Isonitrilen R_{I}-NC abgeleiteten Rest steht, wobei R_{I} ein Isonitrilgruppen tragender linearer oder verzweigter Alkyl- oder Cycloalkylrest aus bis zu 18 C-Atomen, Methoxycarbonylethyl, t-Butoxycarbonylmethyl, Phenyl, o-Alkylphenyl, m-Alkylphenyl, p-Alkylphenyl, o-Halogenphenyl, m-Halogenphenyl, p-Halogenphenyl, 2,3-Dihalogenphenyl, 2,4-Dihalogenphenyl, 3,4-Dihalogenphenyl, o-Alkoxyphenyl, m-Alkoxyphenyl, p-Alkoxyphenyl, o-Arylphenyl, m-Arylphenyl, p-Arylphenyl, o-Aryloxyphenyl, m-Aryloxyphenyl, p-Aryloxyphenyl, o-Nitrophenyl, m-Nitrophenyl, p-Nitrophenyl, 1-Naphthyl, 2-Naphthyl, Benzolsulfonylmethyl, p-Toluolsulfonylmethyl, ein Pyranosyl-, Furanosyl- oder Nucleosyl-Rest, einen n-(Biotinamido)-n-alkylrest mit n = 2-8 oder ein Farbstoffmolekül bedeuten,
und R₁ und R₂ für einen von Carbonylverbindungen abgeleiteten Rest R₁ -C(=O)-R₂ stehen und gleichzeitig oder unabhängig voneinander Wasserstoff, Methyl, Ethyl, Propyl, i-Propyl, Cyclopropyl, Butyl, i-Butyl, t-Butyl, Cyclobutyl, Pentyl, Cyclopentyl, Hexyl, Cyclohexyl, 2-Methylbutyl, Heptyl, Octyl, Nonyl, Decyl, Undecyl, Dodecyl, Methoxycarbonylethyl, t-Butoxycarbonylmethyl, Phenyl, o-Alkylphenyl, m-Alkylphenyl, p-Alkylphenyl, o-Halogenphenyl, m-Halogenphenyl, p-Halogenphenyl, 2,3-Dihalogenphenyl, 2,4-Dihalogenphenyl, 3,4-Dihalogenphenyl, o-Alkoxyphenyl, m-Alkoxyphenyl, p-Alkoxyphenyl, o-Arylphenyl, m-Arylphenyl, p-Arylphenyl, o-Aryloxyphenyl, m-Aryloxyphenyl, p-Aryloxyphenyl, o-Nitrophenyl, m-Nitrophenyl, p-Nitrophenyl, 1-Naphthyl, 2-Naphthyl, Oxiranyl, Vinyl, Propenyl, Propen-2-yl, 2-Penten-2-yl, 3-Hepten-3-yl, Penta-1,3-dienyl, Phenylmethyl, 1-Phenylethyl, 2-Phenylethyl, Cyclohexen-3-yl, Cyclohexen-4-yl, 2-Furyl, 3-Furyl, 2-Thienyl, 3-Thienyl, 2-Pyrrolyl, 3-Pyrrolyl, Halogenmethyl, Dihalogenmethyl, Trihalogenmethyl, 2-Pyridyl, 3-Pyridyl, 4-Pyridyl bedeuten können
und n = 1-15 bedeutet.

2. Verfahren zur Herstellung der Proteinkonjugate nach Anspruch 1, indem man ein lösliches Protein (**P**) oder ein an einer festen Phase immobilisiertes Protein (**P**), das freie Amino-, Carbonsäure- oder Aldehyd-Gruppen trägt, mit einem Amin R_{A}-NH₂ oder einer Carbonsäuren R_{C}-CO₂H, einem Isonitril R_{I}-NC und einer Carbonylverbindung R₁-C(=O)-R₂, wobei die Reste R_{A}, R_{C}, R_{I}, R₁ und R₂ die in Anspruch 1 angegebene Bedeutung haben, in wäßriger Lösung zu den betreffenden Protein-Konjugaten (Ia), (Ib) oder (Ic) umsetzt, wobei die jeweiligen Produkte (Ia) aus einem Protein (**P**), einem Amin R_{A}-NH₂, einem Isonitril R_{I}-NC und einer Carbonylverbindung R₁-C(=O)-R₂, die Produkte (Ib) aus dem Protein (P), einer Carbonsäure R_{C}-CO₂H, einem Isonitril R_{I}-NC und einer Carbonylverbindung R₁- C(=O)-R₂, und die Produkte (Ic) aus einem oxidierten Protein (**P**), einer Carbonsäure R_{C}-CO₂H, einem Amin R_{A}-NH₂ und einem Isonitril R_{I}-NC erhalten werden, wobei die Reste R_{A}, R_{C}, R_{I}, R₁ und R₂ die in Anspruch 1 angegebene Bedeutung haben.

3. Ein Verfahren gemäß Anspruch 2, beim dem die Umsetzung des Proteins (**P**) mit dem Amin R_{A}-NH₂ oder der Carbonsäure R_{C}-CO₂H, und dem Isonitril R_{I}-NC und der Carbonylverbindung R₁-C(=O)-R₂ erfolgt, indem das Protein (**P**) in wäßrigem Puffer gelöst oder suspendiert mit dem Amin R_{A}-NH₂ oder mit der Carbonsäure R_{C}-CO₂H und dem Isonitril R_{I}-NC und der Carbonylverbindung R₁-C(=O)-R₂ umgesetzt, wobei jeweils ein 10-10.000 facher molarer Überschuß zum Protein (**P**) eingesetzt wird.

4. Ein Verfahren gemäß Ansprüchen 2-3, bei dem die Umsetzung in wäßrigen Pufferlösungen, insbesondere in 0.001- 1.0 molaren Lösungen aus Natrium- oder Kalium-dihydrogenphosphat mit Dinatrium- oder Dikalium-hydrogenphosphat, oder in Pufferlösungen aus Tris(hydroxymethyl)-aminomethan mit Salzsäure, im pH-Bereich zwischen 5 und 9 erfolgt.

5. Ein Verfahren gemäß Ansprüchen 2-4, bei dem der Reaktionspuffer weitere Cosolventien wie Methanol, Ethanol, Propanol, i-Propanol, Butanol, Essigsäureethylester, Essigsäuremethylester, Dimethylformamid, Acetonitril, Dimethylsulfoxid oder Sulfolan in Mengen von 0.1-20 Vol-% enthält und die Reaktionstemperaturen zwischen 0° C und 90°C liegen.

6. Die Verwendung der Proteinkonjugate (la-c) nach Anspruch 1 in Immunoassays, synthetische Vaccine oder als Biosensoren.

7. Die therapeutische Verwendung der Proteinkonjugate (la-c) nach Anspruch 1, in denen mindestens einer der Reste R_{C}, R_{A}, R_{I}, R₁ oder R₂ ein Cytostatikum, gebunden an einen Tumor-spezifischen Antikörper, ein Enzym oder ein Lectin darstellt.

8. Die Verwendung des erfindungsgemäßen Verfahrens nach Ansprüchen 2 bis 5 zur Modifizierung von Proteinmischungen in der Probenvorbereitung zur modifizierten Proteomanalyse von Organismen mittels Elektrophorese.
